# EUROPÄISCHE PATENTSCHRIFT

(11) **EP 2 437 595 B1**
(45) Veröffentlichungstag und Bekanntmachung des Hinweises auf die Patenterteilung: **31.10.2018**
(21) Anmeldenummer: 10722006.3
(22) Anmeldetag: 26.05.2010
(51) Int. Cl.: A01N 43/40, A01P 3/00, A01N 43/56

(54) **VERWENDUNG VON FLUOPYRAM ZUR KONTROLLE VON SCLEROTINIA SSP**
USE OF FLUOPYRAM FOR CONTROLLING SCLEROTINIA SSP
UTILISATION D'INHIBITEURS DE FLUOPYRAM POUR LUTTER CONTRE SCLEROTINIA SSP

(30) Priorität: 02.06.2009 EP 09161671
(43) Veröffentlichungstag der Anmeldung: 11.04.2012
(73) Patentinhaber: Bayer CropScience AG, 40789 Monheim am Rhein (DE)
(72) Erfinder: WETCHOLOWSKY, Ingo, 40764 Langenfeld (DE); RIECK, Heiko, 51399 Burscheid (DE); LABOURDETTE, Gilbert, 71600 Paray le Monial (FR); GERALDES, Jose, Augusto, 14015080 Ribeirâo Preto-SP (BR)
(86) Internationale Anmeldenummer: PCT/EP2010/003203
(87) Internationale Veröffentlichungsnummer: WO 2010/139410

(56) Entgegenhaltungen:
- EP-A- 1 389 614
- EP-A- 2 071 953
- EP-A- 2 100 506
- WO-A-03/010149
- WO-A-2006/131221
- WO-A-2007/017231
- WO-A-2007/118069
- JP-A- 2008 133 237

## Beschreibung

### Verwendung von Fluopyram zur Kontrolle von Sclerotinia ssp.

Die Erfindung betrifft die Verwendung von Fluopyram, zur Kontrolle von *Sclerotinia ssp.,* und ein Verfahren zur Behandlung von Sojapflanzen oder Soja-Pflanzenteilen zur Kontrolle von *Sclerotinia* ssp. Sclerotinia ssp. insbesondere *Sclerotinia sclerotiorum* hat 5 bis 20 mm große und fallweise noch größere Sclerotien. Die Pilze überdauern mit Hilfe der Sclerotien im Boden, an befallenen Pflanzenresten oder an perennierenden Unkräutern. Wenn feuchte Bedingungen mehrere Wochen anhalten, kann *Sclerotinia sclerotiorum* das sexuelle Stadium bilden: aus den Sclerotien wachsen 1 bis wenige cm große Apothecien mit Ascosporen. Für die Keimung der Sclerotien müssen Temperaturen zwischen 6 und ca. 15 °C herrschen. Eine Beschattung der Sclerotien und feuchter Boden sind für die Keimung optimal. Die Ascosporen werden schließlich abgeschleudert und können an Blättern und Stielen Infektionen verursachen, vorausgesetzt sie treffen auf geschwächtes Pflanzengewebe oder Wunden. Abgefallene Blütenblätter, die sich in Blattgabeln und Seitentriebachseln verfangen, begünstigen ein Ansiedeln der Sporen und schließlich deren Keimung. Die optimale Temperatur für das Wachstum des Pilzes liegt bei ca. 20°C, er kann aber noch bei 0°C wachsen. Die Sclerotien können bis zu 10 Jahre im Boden überdauern.
Auffallend sind vergilbende Pflanzen, die auch rasch notreif werden. An solchen Pflanzen sieht man am unteren Teil des Haupttrieb stängelumfassend bleiche bis hellbraune Verfärbungen. Das Stängelinnere unter diesen Verfärbungen ist in der Regel hohl, in dem ein weißes, watteartiges Myzel des Pilzes wuchert. Auf diesem Myzel werden kleine schwarze Körner, die Sclerotien, gebildet. Bei hoher Luftfeuchtigkeit oder anhaltend nasser Witterung wird das Myzel und die darauf erscheinenden Sklerotien auch am Stängeläußeren gebildet
*Sclerotinia sclerotiorum* hat große wirtschaftliche Bedeutung neben Raps an der Sonnenblume, an Ackerbohne, Soja, Erbse, Luzerne und den verschiedensten Gemüsekulturen. Auch Unkräuter werden befallen.
*Sclerotinia sclerotiorum* kommt auf fast allen krautigen Kulturpflanzen in gemäßigten Klimazonen vor und ist eines der am meisten gefürchteten Schadpathogene im Sojaanbau.
Es besteht daher ein dringender Bedarf an Fungiziden, die eine ausreichende Kontrolle von *Sclerotinia ssp,* insbesondere von *Sclerotinia sclerotiorum,* in Kulturpflanzen, wie z.B. Raps, Sonnenblume, Ackerbohne, Soja, Erbse, Luzerne und den verschiedensten Gemüsekulturen, ermöglichen. Besonders bevorzugt soll Sclerotinia sclerotiorum in Soja kontrolliert werden.

WO 03/010149 offenbart die Verwendung von Carboxamiden der Formel I zur Bekämpfung von Pilzen wie z.B. Sclerotinia sclerotiorum (S.31, Z.1) auf transgene Pflanzen wie z.B. Soja, Raps (S.44-46). Erfindungsgemäss werden alle Pflanzen, Pflanzenteilen und/oder Vermehrungsmaterial behandelt. Als Mischpartner der obengenannten Carboxamide sind eine Reihe von Fungiziden auf Seiten 36-42 offenbart. Der Lehre der Druckschrift ist jedoch nicht zu entnehmen, welche speziellen Carboxamide zur Behandlung von *Sclerotinia ssp.* geeignet sind.

WO 2006/015865 offenbart Mischungen, enthaltend Succinat Dehydrogenase Inhibitoren wie z.B. Sedaxan und weitere aktive Verbindungen (Ansprüche 1-10) gegen *Sclerotinia spp.* (S.59, Z.7) zur Behandlung von Gras, Soja, Raps, Sonnenblume, Bohnen (S.58, Z.4). Transgene Pflanzen und deren Behandlung sind auf S.51 offenbart. Der Lehre der Druckschrift ist jedoch nicht zu entnehmen, welche speziellen Carboxamide zur Behandlung von *Sclerotinia ssp.* geeignet sind. Insbesondere die Eignung von Sedaxan zur Behandlung von *Sclerotinia ssp.* ist nicht explizit offenbart.

EP-A-1 389614 offenbart Derivate der Pyridinil-Ethyl-Benzamid-Fungizide wie z.B. Fluopyram (Ansprüche 1-15), die gegen Pilze des Genus *Sclerotinia sclerotiorum* (S.6, Z.38-39) auf z.B. Soja-Pflanzen (S.6, Z.4) benutzt werden. Der Lehre der Druckschrift ist jedoch nicht zu entnehmen, welche speziellen Pyridinil-Ethyl-Benzamid-Fungizide zur Behandlung von *Sclerotinia ssp.* geeignet sind. Insbesondere die Eignung von Fluopyram zur Behandlung von *Sclerotinia ssp.* ist nicht explizit offenbart.

WO 2007/1017231 offenbart die Verwendung von Carboxamiden der Formel I (Ansprüche 1-32) zur Saatbehandlung gegen Pilze wie z.B. *Sclerotinia sclerotiorum* in Pflanzen wie z.B. Soja, Raps und Sonnenblume (S.16, Z. 27-30). Als Mischpartner der obengenannten Carboxamide sind eine Reihe von Fungiziden im Anspruch 8 offenbart. Der Lehre der Druckschrift ist jedoch nicht zu entnehmen, welche speziellen Carboxamiden der Formel I zur Behandlung von *Sclerotinia ssp.* geeignet sind.

WO 2006/131221 offenbart die Verwendung von Carboxamiden der Formel I wie z.B. die Succinat Dehydrogenase Inhibitoren Boscalid und Penthiopyrad (Anspruch 4) zur Bekämpfung von Rostpilzen wie z.B. *Sclerotinia sclerotiorum* auf Soja-Pflanzen (S.28, Z.29 bis S29, Z.12). Transgene Pflanzen, die behandelt werden können wie z.B. Soja-Pflanzen sind ebenso offenbart (Abs. 2, S.37, Ansp.6). Saatgutbehandlung ist im Abs. 2, S. 36 offenbart. Als Mischpartner der obengenannten Carboxamide sind eine Reihe von Fungiziden auf Seiten31-32 offenbart. Der Lehre der Druckschrift ist jedoch nicht zu entnehmen, welche speziellen Carboxamiden der Formel I zur Behandlung von *Sclerotinia ssp.* geeignet sind. Insbesondere ist die Eignung von Boscalid oder Penthiopyrad zur Behandlung von *Sclerotinia ssp.* nicht explizit offenbart.
WO 2007/118069 offenbart ein Verfahren zur Behandlung vom Gras oder Grassaatgut gegen Pilze wie z.B. *Sclerotinia ssp.* (Ansprüche 11-15) mittels aktiver Carboxamide der Formel I (z.B. Isopyrazam). Als Mischpartner der obengenannten Carboxamide sind eine Reihe von Fungiziden auf Seiten 19-20 offenbart. Auch dieser Druckschrift ist jedoch nicht zu entnehmen, welche speziellen Carboxamiden der Formel I zur Behandlung von *Sclerotinia ssp.* geeignet sind.
JP 2008/133237 offenbart ein Verfahren zur Bodenbehandlung bei Pflanzen z.B. Bohnen gegen Pilze der Art *Sclerotinia sclerotiorum* [0007] mittels Pyrazolcarboxamide wie z.B. Penthiopyrad [0001].
EP-A 2 100 506 beschreibt allgemein die Verwendung von Fluopyram allein oder in Kombination mit bestimmten anderen Wirkstoffen gegen Sclerotinia in Raps und Bohnen.
Überraschenderweise wurde nun gefunden, dass der Succinat Dehydrogenase Inhibitor Fluopyram hervorragend zur Kontrolle von *Sclerotinia ssp,* insbesondere von *Sclerotinia sclerotiorum,* in Sojapflanzen geeignet ist.

Besonders vorteilhaft hat sich die Verwendung von Fluopyram zur Kontrolle von *Sclerotinia sclerotiorum* in Soja erwiesen.

Gegenstand der vorliegenden Erfindung ist demnach die Verwendung von Fluopyram zur Kontrolle von bevorzugt *Sclerotinia sclerotiorum.*

Fluopyram mit dem chemischen Namen N-{[3-chlor-5-(trifluormethyl)-2-pyridinyl]ethyl}-2,6-dichlorbenzamide und geeignete Verfahren zu dessen Herstellung sind, ausgehend von kommerziell zugänglichen Ausgangsstoffen, in EP-A- 1 389 614 beschrieben.
Im Zusammenhang mit der vorliegenden Erfindung bedeutet "Kontrolle von *Sclerotinia ssp*." eine signifikante Verminderung des Befalls durch *Sclerotinia ssp.,* verglichen mit der unbehandelten Pflanze, vorzugsweise eine wesentliche Verminderung (um 40-79 %), verglichen mit der unbehandelten Pflanze (100%), besonders bevorzugt wird die Infektion durch *Sclerotinia ssp.* vollständig unterdrückt (um 70-100%). Die Kontrolle kann dabei kurativ, d.h. zur Behandlung bereits infizierter Pflanzen oder protektiv, d.h. zum Schutz bislang nicht infizierter Pflanzen erfolgen.

Insbesondere zeigt die erfindungsgemäße Verwendung in der Sprühapplikation, bei Drip und Drench Applikationen, Chemigation, d.h. durch Zugabe der Wirkstoffe zum Bewässerungswasser, und in hydroponischen/mineralischen Systemen auf Pflanzen und Pflanzenteile die beschriebenen Vorteile.

Kombinationen von Fluopyram mit einem weiteren fungiziden Wirkstoff können bei der Bekämpfung von Pflanzenkrankheiten im Rahmen der vorliegenden Erfindung ebenfalls Anwendung finden. Die kombinierte Verwendung von Fluopyram mit gentechnisch veränderten Sorten, insbesondere von transgenen Sojasorten ist darüber hinaus ebenfalls möglich.

Im Rahmen der vorliegenden Erfindung wird unter einer Pflanze eine Pflanze ab dem Stadium der Blattentwicklung verstanden (ab dem Stadium BBCH 10 gemäß der BBCH-Monografie der Biologische Bundesanstalt für Land und Forstwirtschaft, 2. Auflage, 2001).

In einer Ausführungsform kann beispielsweise vorgesehen sein, dass Fluopyram durch eine Sprühapplikation auf entsprechende zu behandelnde Pflanzen oder Pflanzenteile aufgebracht wird. Die erfindungsgemäß vorgesehene Verwendung des Succinat Dehydrogenase Inhibitors Fluopyram erfolgt mit einer Dosierung zwischen 0,1 und 1 kg/ha.
Erfindungsgemäß kann der Succinat Dehydrogenase Inhibitor Fluopyram in Abhängigkeit von seinem jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.
Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.
Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.
Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.
Die Formulierungen enthalten im Allgemeinen zwischen 0,1 und 95 Gewichtsprozent Wirkstoff, vorzugsweise zwischen 0,5 und 90 %.

Unter Pflanzensorten versteht man Pflanzen mit neuen Eigenschaften ("Traits"), die sowohl durch konventionelle Züchtung, durch Mutagenese oder mit Hilfe rekombinanter DNA-Techniken, gezüchtet worden sind. Kulturpflanzen können demnach Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten.
Das erfindungsgemäße Verfahren kann somit auch für die Behandlung von genetisch modifizierten Organismen (GMOs), z. B. Pflanzen verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dasses ein anderes Gen, das in der Pflanze vorliegt bzw. andere Gene, die in der Pflanze vorliegen, herunterreguliert oder abschaltet (zum Beispiel mittels Antisense-Technologie, Co-suppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.
Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde). Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Hybridpflanzen sind Pflanzen, die bereits die Eigenschaft der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben (WO 1992/005251, WO 1995/009910, WO 1998/27806, WO 2005/002324, WO 2006/021972 und US 6,229,072). Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden (z. B. WO 1991/002069).

Pflanzen oder Pflanzensorten, die mit Methoden der Pflanzenbiotechnologie, wie der Gentechnik, erhalten wurden, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.
Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., Science (1983), 221, 370-371), das CP4-Gen des Bakteriums Agrobacterium sp. (Barry et al., Curr. Topics Plant Physiol. (1992), 7, 139-145), die Gene, die für eine EPSPS aus der Petunie (Shah et al., Science (1986), 233, 478-481), für eine EPSPS aus der Tomate (Gasser et al., J. Biol. Chem. (1988), 263, 4280-4289) oder für eine EPSPS aus Eleusine (WO 2001/66704) kodieren. Es kann sich auch um eine mutierte EPSPS handeln, wie sie zum Beispiel in EP-A 0837944, WO 2000/066746, WO 2000/066747 oder WO 2002/026995 beschrieben ist. Glyphosatetolerante Pflanzen können auch dadurch erhalten werden, dass man ein Gen exprimiert, das für ein Glyphosate-Oxidoreduktase-Enzym, wie es in US 5,776,760 und US 5,463,175 beschrieben ist, kodiert. Glyphosatetolerante Pflanzen können auch dadurch erhalten werden, dass man ein Gen exprimiert, das für ein Glyphosate-acetyltransferase-Enzym, wie es in z. B. WO 2002/036782, WO 2003/092360, WO 2005/012515 und WO 2007/024782 beschrieben ist, kodiert. Glyphosatetolerante Pflanzen können auch dadurch erhalten werden, dass man Pflanzen, die natürlich vorkommende Mutationen der oben erwähnten Gene, wie sie zum Beispiel in WO 2001/024615 oder WO 2003/013226 beschrieben sind, enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind zum Beispiel in US 5,561,236; US 5,648,477; US 5,646,024; US 5,273,894; US 5,637,489; US 5,276,268; US 5,739,082; US 5,908,810 und US 7,112,665 beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym gemäß WO 1996/038567, WO 1999/024585 und WO 1999/024586 kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen und Gene sind in WO 1999/034008 und WO 2002/36787 beschrieben. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert, wie dies in WO 2004/024928 beschrieben ist.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen, wie dies zum Beispiel bei Tranel und Wright, Weed Science (2002), 50, 700-712, jedoch auch in US 5,605,011, US 5,378,824, US 5,141,870 und US 5,013,659, beschrieben ist. Die Herstellung von sulfonylharnstofftoleranten Pflanzen und imidazolinontoleranten Pflanzen ist in US 5,605,011; US 5,013,659; US 5,141,870; US 5,767,361; US 5,731,180; US 5,304,732; US 4,761,373; US 5,331,107; US 5,928,937; und US 5,378,824; sowie in der internationalen Veröffentlichung WO 1996/033270 beschrieben. Weitere imidazolinontolerante Pflanzen sind auch in z. B. WO 2004/040012, WO 2004/106529, WO 2005/020673, WO 2005/093093, WO 2006/007373, WO 2006/015376, WO 2006/024351 und WO 2006/060634 beschrieben. Weitere sulfonylharnstoff- und imidazolinontolerante Pflanzen sind auch in z.B. WO 2007/024782 beschrieben.
Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden, wie dies zum Beispiel für die Sojabohne in US 5,084,082, für Reis in WO 1997/41218, für die Zuckerrübe in US 5,773,702 und WO 1999/057965, für Salat in US 5,198,599 oder für die Sonnenblume in WO 2001/065922 beschrieben ist.

Pflanzen oder Pflanzensorten, die nach Methoden der pflanzlichen Biotechnologie, wie der Gentechnik, erhalten wurden, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst jegliche Pflanze, die mindestens ein Transgen enthält, das eine Kodiersequenz umfasst, die für folgendes kodiert:
1) ein insektizides Kristallprotein aus Bacillus thuringiensis oder einen insektiziden Teil davon, wie die insektiziden Kristallproteine, die von Crickmore et al., Microbiology and Molecular Biology Reviews (1998), 62, 807-813, von Crickmore et al. (2005) in der Bacillus thuringiensis-Toxinnomenklatur aktualisiert, online bei: http://www.lifesci.sussex.ac.uk/Home/Neil_Crickmore/Bt/), zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Moellenbeck et al., Nat. Biotechnol. (2001), 19, 668-72; Schnepf et al., Applied Environm. Microb. (2006), 71, 1765-1774); oder
3) ein insektizides Hybridprotein, das Teile von zwei unterschiedlichen insektiziden Kristallproteinen aus Bacillus thuringiensis umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bbl in Mais-Events MON863 oder MON88017 oder das Protein Cry3A im Mais-Event MIR 604; oder
5) ein insektizides sezerniertes Protein aus Bacillus thuringiensis oder Bacillus cereus oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 1994/21795); oder
7) ein insektizides Hybridprotein, das Teile von verschiedenen sezernierten Proteinen von Bacillus thuringiensis oder Bacillus cereus umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten, die nach Methoden der pflanzlichen Biotechnologie, wie der Gentechnik, erhalten wurden, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag, wie dies in WO 2000/004173 oder EP 04077984.5 oder EP 06009836.5 beschrieben ist.
b. Pflanzen, die ein Stresstoleranz-förderndes Transgen enthalten, das die Expression und/oder Aktivität der für PARG kodierenden Gene der Pflanzen oder Pflanzenzellen zu reduzieren vermag, wie dies z.B. in WO 2004/090140 beschrieben ist;
c. Pflanzen, die ein Stresstoleranz-förderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase, wie dies z. B. in EP 04077624.7 oder WO 2006/133827 oder PCT/EP07/002433 beschrieben ist.

Pflanzen oder Pflanzensorten, die nach Methoden der pflanzlichen Biotechnologie, wie der Gentechnik, erhalten wurden, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet. Diese transgenen Pflanzen, die eine modifizierte Stärke synthetisieren, sind zum Beispiel in EP 0571427, WO 1995/004826, EP 0719338, WO 1996/15248, WO 1996/19581, WO 1996/27674, WO 1997/11188, WO 1997/26362, WO 1997/32985, WO 1997/42328, WO 1997/44472, WO 1997/45545, WO 1998/27212, WO 1998/40503, WO 99/58688, WO 1999/58690, WO 1999/58654, WO 2000/008184, WO 2000/008185, WO 2000/28052, WO 2000/77229, WO 2001/12782, WO 2001/12826, WO 2002/101059, WO 2003/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 2000/22140, WO 2006/063862, WO 2006/072603, WO 2002/034923, EP 06090134.5, EP 06090228.5, EP 06090227.7, EP 07090007.1, EP 07090009.7, WO 2001/14569, WO 2002/79410, WO 2003/33540, WO 2004/078983, WO 2001/19975, WO 1995/26407, WO 1996/34968, WO 1998/20145, WO 1999/12950, WO 1999/66050, WO 1999/53072, US 6,734,341, WO 2000/11192, WO 1998/22604, WO 1998/32326, WO 2001/98509, WO 2001/98509, WO 2005/002359, US 5,824,790, US 6,013,861, WO 1994/004693, WO 1994/009144, WO 1994/11520, WO 1995/35026 bzw. WO 1997/20936 beschrieben.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, wie dies in EP 0663956, WO 1996/001904, WO 1996/021023, WO 1998/039460 und WO 1999/024593 beschrieben ist, Pflanzen, die alpha-1,4-Glucane produzieren, wie dies in WO 1995/031553, US 2002/031826, US 6,284,479, US 5,712,107, WO 1997/047806, WO 1997/047807, WO 1997/047808 und WO 2000/14249 beschrieben ist, Pflanzen, die alpha-1,6-verzweigte alpha-1,4-Glucane produzieren, wie dies in WO 2000/73422 beschrieben ist, und Pflanzen, die Alternan produzieren, wie dies in WO 2000/047727, EP 06077301.7, US 5,908,975 und EP 0728213 beschrieben ist.
3) Transgene Pflanzen, die Hyaluronan produzieren, wie dies zum Beispiel in WO 2006/032538, WO 2007/039314, WO 2007/039315, WO 2007/039316, JP 2006/304779 und WO 2005/012529 beschrieben ist.

Pflanzen oder Pflanzensorten, die nach Methoden der pflanzlichen Biotechnologie, wie der Gentechnik, erhalten wurden, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten, wie dies in WO 1998/000549 beschrieben ist,
b) Pflanzen wie Baumwollpflanzen, die eine veränderte Form von rsw2- oder rsw3-homologen Nukleinsäuren enthalten, wie dies in WO 2004/053219 beschrieben ist;
c) Pflanzen wie Baumwollpflanzen mit einer erhöhten Expression der Saccharosephosphatsynthase, wie dies in WO 2001/017333 beschrieben ist;
d) Pflanzen wie Baumwollpflanzen mit einer erhöhten Expression der Saccharosesynthase, wie dies in WO 02/45485 beschrieben ist;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase, wie dies in WO 2005/017157 beschrieben ist;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen, wie dies in WO 2006/136351 beschrieben ist.

Pflanzen oder Pflanzensorten, die nach Methoden der pflanzlichen Biotechnologie, wie der Gentechnik, erhalten wurden, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren, wie dies zum Beispiel in US 5,969,169, US 5,840,946 oder US 6,323,392 oder US 6,063, 947 beschrieben ist;
b) Pflanzen wie Rapspflanzen, die Öl mit einem niedrigen Linolensäuregehalt produzieren, wie dies in US 6,270828, US 6,169,190 oder US 5,965,755 beschrieben ist.
c) Pflanzen wie Rapspflanzen, die Öl mit einem niedrigen gesättigten Fettsäuregehalt produzieren, wie dies z. B. in US 5,434,283 beschrieben ist.

Besonders nützliche transgene Pflanzen, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).
Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/ dbase.php).

### Formulierungen:

Der erfindungsgemäße Succinat Dehydrogenase Inhibitor Fluopyram kann in seinen handelsüblichen Formulierungen sowie in den aus diesen Formulierungen bereiteten Anwendungsformen in Mischung mit einem weiteren fungiziden Wirkstoff vorliegen.
Ferner lässt sich die beschriebene positive Wirkung des Succinat Dehydrogenase Inhibitors Fluopyram auf die Kontrolle von *Sclerotinia ssp.* durch eine zusätzliche Behandlung mit insektziden, fungiziden oder bakteriziden Wirkstoffen unterstützen.
Bevorzugte Zeitpunkte für die Applikation von Azolverbindungen zur Steigerung der Resistenz gegenüber abiotischem Stress sind Boden-, Stamm- und/oder Blattbehandlungen mit den zugelassenen Aufwandmengen.

Der erfindungsgemäße Succinat Dehydrogenase Inhibitor Fluopyram kann im Allgemeinen darüber hinaus in seinen handelsüblichen Formulierungen vorliegen.

Das folgende Beispiel dient der Verdeutlichung der Erfindung.

### Beispiel 1

In Brasilien wurde ein Parzellenversuch mit der Sojasorte Monsoy 7908 RR angelegt, um die Wirkung von Fluopyram gegen *Sclerotinia sclerotiorum* in der Spritzanwendung unter landwirtschaftlichen Praxisbedingungen zu überprüfen.

Die zu prüfenden Produkte wurden in Form von sequentiellen Spritzfolgen auf die Pflanzen gespritzt.

Die Zeiträume zwischen den einzelnen Spritzungen variierten zwischen 2 - 3 Wochen.

Fluopyram wurde in einer Formulierung 500 SC (Formulierungsnummer SP 102000016460) mit Aufwandmengen von 150, 200 und 250 g a.i./ha angewendet. Das Spritzvolumen betrug 300 1 Wasser je Hektar. 30 Tage nach der dritten Spritzung wurde der Bekämpfungserfolg visuell in den Parzellen ausgewertet. Die Bonitur erfolgte durch Zählung der befallenen Pflanzen in Teilstücken der Parzelle. Der Wirkungsgrad wurde danach gemäß der Abbottformel errechnet.

Die Befallszahlen sind in der folgenden Tabelle 1 zu entnehmen:

**Tab.1 Wirkung von Fluopyram gegen Sclerotinia sclerotiorum in Soja**

| **Tabelle 1** | | | |
|---|---|---|---|
| **Behandlungsvariante** | **Dosis Wirkstoff (g a.i./ha)** | **Anzahl befallener Pflanzen 30 Tage nach der dritten Spritzung** | **Wirkungsgrad (% Abbott)** |
| Unbehandelte Kontrolle | | 17 | |
| Fluopyram | 150 | 2,7 | 84 |
| Fluopyram | 200 | 1,7 | 90 |
| Fluopyram | 250 | 0,3 | 98 |

## Patentansprüche

1. Verwendung von Fluopyram in einer Anwendungsrate von 0.1 bis 1 kg/ha zur Kontrolle von *Sclerotinia ssp* in Sojapflanzen, wobei die Pflanze eine Pflanze ab BBCH-Stadium 10 ist.

2. Verwendung gemäß Anspruch 1, wobei die *Sclerotinia* Spezies *Sclerotinia sclerotiorum* ist.

3. Verwendung nach einem der Ansprüche 1 oder 2 , **dadurch gekennzeichnet, dass** die Pflanzen transgene Pflanzen sind.

4. Verwendung nach einem der Ansprüche 1 bis 3 , **dadurch gekennzeichnet, dass** Fluopyram in Kombination mit einem weiteren fungiziden Wirkstoff angewendet wird.

5. Verfahren zur Kontrolle von *Sclerotinia ssp.* in Sojapflanzen oder Soja-Pflanzenteilen, **dadurch gekennzeichnet, dass** die Sojapflanzen oder Soja-Pflanzenteile mit Fluopyram in einer Anwendungsrate von 0.1 bis 1 kg/ha behandelt werden und die Pflanzen ab BBCH-Stadium 10 sind.

## Claims

1. Use of fluopyram at an application rate of 0.1 to 1 kg/ha for control of *Sclerotinia ssp* in soya plants, wherein the plant is a plant at or after BBCH stage 10.

2. Use according to Claim 1, where the *Sclerotinia* species is *Sclerotinia sclerotiorum.*

3. Use according to either of Claims 1 and 2, **characterized in that** the plants are transgenic plants.

4. Use according to any of Claims 1 to 4, **characterized in that** fluopyram is employed in combination with a further active fungicidal ingredient.

5. Method for controlling *Sclerotinia ssp.* in soya plants or soya plant parts, **characterized in that** the soya plants or soya plant parts are treated with fluopyram at an application rate of 0.1 to 1 kg/ha and the plants are at or after BBCH stage 10.

## Revendications

1. Utilisation de fluopyram en un taux d'application de 0,1 à 1 kg/ha pour l'élimination de *Sclerotinia ssp* dans des plantes de soja, la plante étant une plante à partir du stade BBCH 10.

2. Utilisation selon la revendication 1, dans laquelle l'espèce *Sclerotinia* est *Sclerotinia sclerotiorum.*

3. Utilisation selon l'une quelconque des revendications 1 ou 2, **caractérisée en ce que** les plantes sont des plantes transgéniques.

4. Utilisation selon l'une quelconque des revendications 1 à 3, **caractérisée en ce que** le fluopyram est utilisé en combinaison avec un autre agent actif fongicide.

5. Procédé d'élimination de *Sclerotinia ssp.* dans des plantes de soja ou des parties de plantes de soja, **caractérisé en ce que** les plantes de soja ou les parties de plantes de soja sont traitées avec du fluopyram en un taux d'application de 0,1 à 1 kg/ha, et les plantes sont à partir du stade BBCH 10.
